# EUROPEAN PATENT SPECIFICATION

(11) **EP 1 638 522 B1**
(45) Date of publication and mention of the grant of the patent: **12.01.2011**
(21) Application number: 04750642.3
(22) Date of filing: 26.04.2004
(51) Int. Cl.: A61K 9/00, A61K 38/09

(54) **SOLID DRUG FORMULATION AND DEVICE FOR STORAGE AND CONTROLLED DELIVERY THEREOF**
FESTE ARZNEIMITTELFORMULIERUNG UND VORRICHTUNG ZU IHRER AUFBEWAHRUNG UND KONTROLLIERTEN ABGABE
FORMULATION DE MEDICAMENT SOLIDE, ET DISPOSITIF DE STOCKAGE ET DE DISTRIBUTION CONTROLEE DE CE MEDICAMENT

(30) Priority: 25.04.2003 US 465466 P
(43) Date of publication of application: 29.03.2006
(73) Proprietor: Boston Scientific Scimed, Inc., Maple Grove, MN 55311-1566 (US)
(72) Inventor: PRESCOTT, James, H., Cambridge, MA 02138 (US); UHLAND, Scott, A., Roslindale, MA 02131 (US); STAPLES, Mark, A., Cambridge, MA 02142 (US); SANTINI, John, T., Jr, North Chelmsford, MA 01863 (US)
(74) Representative: Peterreins, Frank
(86) International application number: PCT/US2004/012757
(87) International publication number: WO 2004/096176

(56) References cited:
- WO-A-2004/022033
- WO-A-2004/033036
- US-A- 4 917 685
- US-A- 5 981 489
- US-A1- 2002 107 470
- US-B1- 6 264 990
- SANTINI J T ET AL: "MICROCHIPS AS CONTROLLED DRUG-DELIVERY DEVICES" ANGEWANDTE CHEMIE. INTERNATIONAL EDITION, VERLAG CHEMIE. WEINHEIM, DE, vol. 39, no. 14, 17 July 2000 (2000-07-17), pages 2397-2407, XP000954590 ISSN: 0570-0833

## Description

### Background of the Invention

This invention is generally in the field of methods and compositions for use in the delivery of a drug to patients, and more particularly to stabilized drug formulations comprising solid forms of protein or other types of active agents. The invention also relates to methods for the controlled handling and storage of unstable proteins or other molecules and the improved production, filling, and storage of dry forms of such molecules.

Many useful proteins and other molecules that are unstable in aqueous solutions are handled and stored as dry solids ("dry" is defined within this document as substantially free of residual moisture, typically with a water content not exceeding 10% w/w). Bulk drying and lyophilization (freeze-drying) are known useful ways to stabilize protein structure and activity. Traditional freeze-drying methods involve the freezing of an aqueous solution containing various stabilizing agents, followed by application of a vacuum to remove the water by sublimation, producing a dry porous solid that is relatively stable and suitable for long-term storage.

Dry solids (particularly powders) are frequently sensitive to packing forces, static charge, moisture, and other variables that can affect the handling of the powder, making it difficult to reproduce or deliver precise quantities, particularly microquantities, of the powders. For example, it could be difficult to control the predictability or repeatability of release characteristics of the powder from a drug delivery device. It therefore would be advantageous to minimize or eliminate such difficulties. It therefore would be desirable to provide improved methods for storing and releasing stable, dry solid forms of proteins and other active agents, particularly from microscale reservoirs containing a pharmaceutical formulation.

In addition and more generally, it would be desirable to provide compositions and methods to precisely handle and process, stably store, and accurately deliver drug formulations, particularly proteins and peptides at high concentrations.

### Summary of the Invention

In one aspect, a device is provided for the storage and controlled release of a solid form of a drug. In one embodiment, this device comprises a body portion; one or more reservoirs located in and defined by the body portion; a porous solid monolithic matrix which comprises a drug and which is contained in each of the one or more reservoirs; and one or more excipient materials dispersed throughout pores within the solid matrix and substantially filling any space not otherwise occupied by the solid matrix within each of the one or more reservoirs, wherein the excipient material enhances stability of the drug while stored in the one or more reservoirs or enhances release of the drug from each reservoir.

In various embodiments, at least one of the one or more excipient materials is in a solid, liquid, semi-solid, or gel state at ambient conditions.

In one embodiment, the one or more excipient materials are non-aqueous. For example, the excipient material can comprises a polymer, such as a polyethylene glycol. In one embodiment, the polyethylene glycol has a molecular weight between about 100 and 10,000 Da. In another embodiment, at least one of the one or more excipient materials comprises a perhalohydrocarbon or unsubstituted saturated hydrocarbon. In yet another embodiment, at least one of the one or more excipient materials comprises dimethyl sulfoxide or ethanol. In a further embodiment, at least one of the one or more excipient materials comprises a pharmaceutically-acceptable oil. In still a further embodiment, the excipient material comprises a saturated solution of the drug.

In one embodiment, the drug comprises an amino acid, a peptide, or a protein. In various embodiments, the drug is selected from glycoproteins, enzymes, hormones, interferons, interleukins, and antibodies. For example, the drug can comprise a human parathyroid hormone, a leutenizing hormone-releasing hormone, a gonadotropin-releasing hormone, or an analog thereof. In yet another embodiment, the drug comprises a natriuretic peptide.

In one embodiment, the one or more reservoirs are microreservoirs. For example, the volume of each reservoir is between 10 nL and 500 nL in one particular embodiment. In another embodiment, each of the one or more reservoirs has a volume between 10 µL and 500 µL.

The body portion can take a variety of forms. In various embodiments, the body portion is in the form of a chip, a disk, a tube, a sphere, or a stent. The body portion can comprise, for example, silicon, a metal, a ceramic, a polymer, or a combination thereof.

In one preferred embodiment, the device comprises a plurality of the reservoirs located in discrete positions across at least one surface of the body portion. In one embodiment, each reservoir has an opening covered by an impermeable reservoir cap which can be selectively ruptured to initiate release of the drug from the reservoir.

In one embodiment, a first excipient material is dispersed throughout pores or interstices within the solid matrix and a second excipient material substantially fills reservoir space not occupied by the first excipient material within each of the one or more reservoirs.

In a preferred embodiment, the one or more excipient materials, upon exposure to an environmental solvent (e.g., a physiological fluid) for the drug, promote dissolution of the drug to enhance release of the drug from the reservoir. In one embodiment, the one or more excipient materials prevent aggregation or precipitation of the drug upon exposure to an environmental fluid to enhance release of the drug from the reservoir.

In one embodiment, the device is adapted for implantation into a patient, and the excipient material comprises an organic solvent. Preferably, the device releases *in vivo* an amount of the organic solvent that is less than the predetermined maximum daily exposure for the organic solvent.

In another aspect, a method is provided for making a device for the storage and controlled release of a solid form of a drug. In one embodiment, the method comprises: providing a drug in dry, porous matrix form; and combining with the drug matrix at least one excipient material which substantially fills the pores and interstices within the matrix to form a drug/excipient composite, wherein the drug/excipient composite, alone or in combination with another excipient material, substantially fills each of one or more reservoirs located in a body portion of a device for the storage and controlled release of the drug.

In one embodiment, the dry, porous matrix form of the drug is first provided in the one or more reservoirs and then fluidized excipient material is added to the one or more reservoirs. In one embodiment, the method further comprises solidifying the fluidized excipient material.

In one embodiment, the dry, porous matrix form of the drug is formed by a method comprising: dissolving or dispersing a drug in a volatile liquid medium to form a first fluid; depositing a quantity of the first fluid into each of one or more reservoirs; and drying the quantity by volatilizing the volatile liquid medium to produce the dry, porous matrix of the drug in the one or more reservoirs.

In another embodiment, the at least one excipient material is in a molten state when combined with the drug matrix.

In yet another embodiment, the dry porous matrix form of the drug and the at least one excipient material first are combined together outside of the one or more reservoirs to form a drug/excipient composite and then the drug/excipient composite is loaded into the one or more reservoirs. For example, the drug/excipient composite can be solidified into a pre-form before being loaded into the one or more reservoirs, each pre-form being shaped to fit into and substantially fill one of the one or more reservoirs. In another example, the drug/excipient composite is melt-extruded into the reservoirs.

In another aspect, a pharmaceutical composition is provided which comprises a solid matrix which comprises a drug, and one or more excipient materials dispersed throughout pores or interstices within the solid matrix, wherein the excipient material enhances stability of the drug while stored and subsequent dissolution upon administration. In one embodiment, the composition is in the form of a plurality of discrete pellets.

In yet another aspect, a sensor device is provided, which comprises a body portion; one or more reservoirs located in and defined by the body portion; a solid matrix which comprises a sensor material and which is contained in each of the one or more reservoirs; and one or more excipient materials substantially filling any space not otherwise occupied by the solid matrix within each of the one or more reservoirs, to eliminate gas pockets in the reservoir.

### Brief Description of the Drawings

FIG. 1 is a perspective, cross-sectional view of one embodiment of the reservoir and body portion of the drug delivery device described herein.
FIG. 2 illustrates one embodiment of the process steps for loading a reservoir with the a solid drug matrix and backfilling with an excipient material.
FIG. 3 is a graph of normalized leuprolide recovery over time for various formulations comprising solid form leuprolide.
FIGS. 4A-B are exterior and interior perspective views, respectively, of one embodiment of an implantable drug delivery device which can be loaded with the drug formulations described herein.
FIG. 5 is an exterior perspective view of another embodiment of an implantable drug delivery device which can be loaded with the drug formulations described herein.

### Detailed Description of the Invention

Methods have been developed for formulating a solid form of a drug for controlled release from a containment device, such as a microchip device comprising an array of micro-reservoirs. Implantable drug delivery devices loaded with these formulations are provided.

It had been observed that the *in vitro* release of a lyophilized drug from small reservoirs can be inhibited by the presence of air bubbles in the reservoir. While not being limited to any theory, it is believed that these bubbles result from the void spaces in the solid drug and prevent fluid from outside the reservoir from entering the reservoir and contacting the solid drug, thereby inhibiting dissolution of the drug and diffusion of the dissolved drug out of the reservoir. It was discovered that the use of a void-displacing excipient with the solid drug in the containment device could provide greater control of drug release properties (kinetics) than would occur in the absence of the void-displacing excipient. For example, the methods and improved formulations can help keep the solid active pharmaceutical ingredient stable during storage in the containment device, can prevent air bubbles from hindering release of the drug from the containment device, and/or can enhance redissolution of the drug upon release/administration to a patient in need thereof.

The methods involve providing a drug in dry, porous matrix form, and then adding to the drug matrix an excipient material that substantially fills the pores and interstices within the matrix. These formulations can be made, stored, and used in a variety of devices and drug delivery systems. The composition is particularly useful in drug delivery devices having small reservoir openings through which the drug is released. The excipient may solidify or remain liquid following loading of the formulation into the device reservoirs. Having the excipient material in the pores of the matrix enhances the stability and/or redissolution of the drug by keeping the local concentration of the drug lower during the redissolution process as compared to the concentration if no excipient material were included, thereby avoiding or minimizing having the local concentration of the drug during redissolution exceed the solubility of the drug and cause reprecipitation, which could block the reservoir opening, and/or minimizing unacceptable aggregation of peptide or protein drug molecules.

These reservoir loading and formulation methods can also be adapted for use in sensor applications, for example where the reservoirs are loaded with a chemical-based sensor instead of a drug.

As used herein, the terms "comprise," "comprising," "include," and "including" are intended to be open, non-limiting terms, unless the contrary is expressly indicated. **I. Devices for Stowage and Release/Exposure of a Solid Drug or Sensor**

### Device for Storage and Delivery of Drug

In one aspect, a device is provided for the storage and delivery of a solid form drug formulation to a patient in need thereof. In one embodiment, the device for the storage and controlled release of a solid form of a drug comprises a body portion; one or more reservoirs located in and defined by the body portion; a solid matrix which comprises a drug and which is contained in each of the one or more reservoirs; and one or more excipient materials dispersed throughout pores or interstices within the solid matrix and substantially filling any space not otherwise occupied by the solid matrix within each of the one or more reservoirs, wherein the excipient material enhances stability of the drug while stored in the one or more reservoirs or enhances release of the drug from each reservoir.

As used herein, the terms "substantially fill" and "substantially filling" refers to filling the void volume of the solid drug matrix and/or of the reservoir with at least an amount of excipient material sufficient to improve dissolution/release characteristics of the drug formulation as compared to that of solid drug matrix without the excipient material present in the pores and interstices of the drug matrix and reservoir spaces.

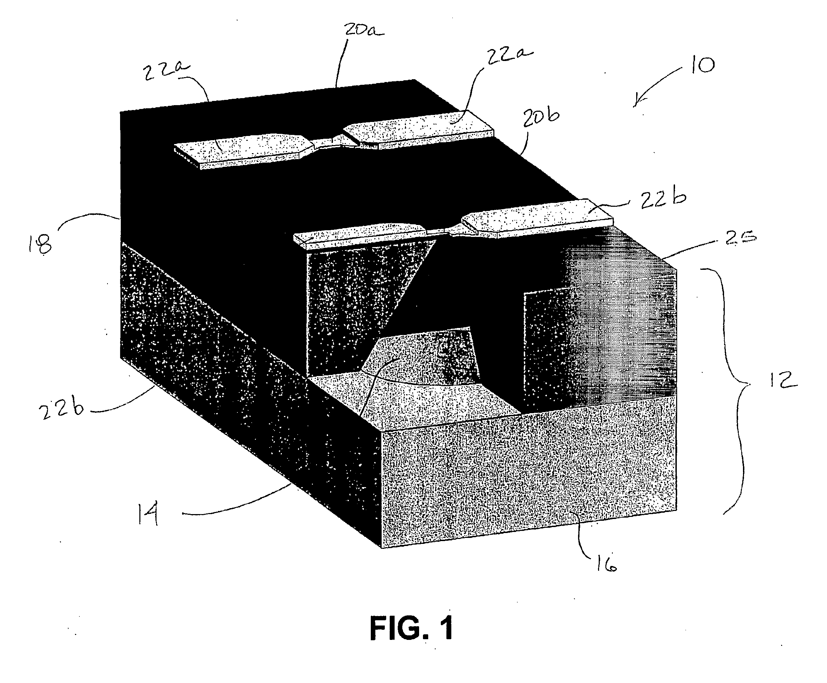

In one embodiment, each reservoir has an opening covered by a reservoir cap that can be selectively ruptured (e.g., disintegrated) to initiate release of the drug from the reservoir. In a preferred embodiment, the reservoir cap comprises a metal film and is disintegrated by electrothermal ablation as described in U.S. Serial No. 10/641,507, filed August 15, 2003. This embodiment is illustrated in **FIG. 1****,** which shows device **10** (shown only in part) which comprises body portion **12,** which includes a first substrate portion **18** and a second substrate portion **16.** Reservoirs **14** are defined in the body portion. (Two are located in the body portion in this illustration, but only one can be seen from the cut-away of part of the first substrate portion.) The release opening of the reservoirs are covered by reservoir caps **20a** and **20b.** Metal conductors **22a** and **22b** are electrically connected to the reservoir caps, for delivering electric current to the reservoir caps. Dielectric layer **25** is provided on the outer surface of the first substrate portion and is underneath the conductors.

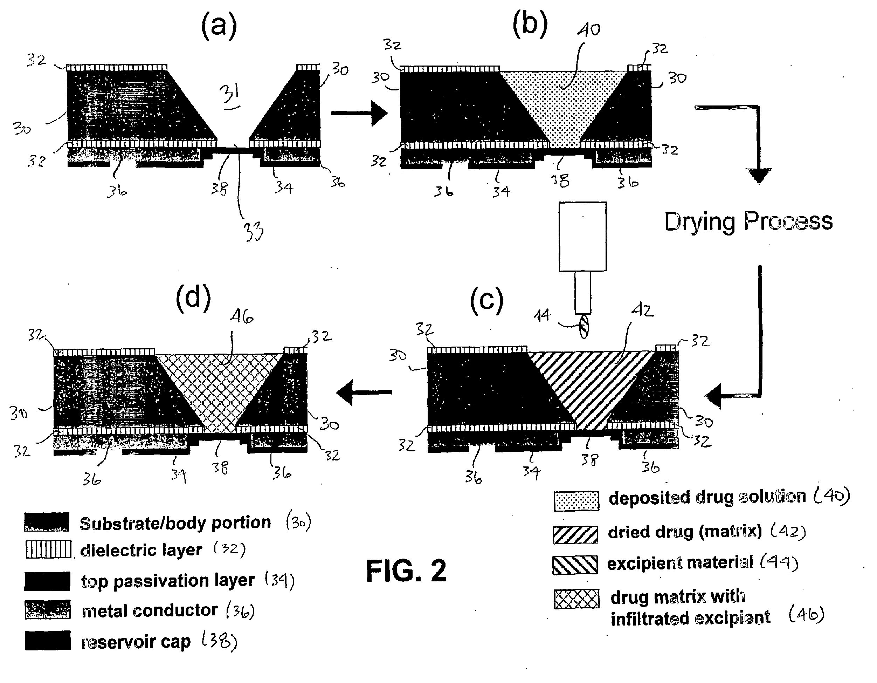

**FIG. 2** shows in a cross-sectional view one embodiment of a reservoir in the body portion and shows the reservoir being loaded with the drug formulation described herein. The substrate **30** includes reservoir **31,** which has release opening **33** covered by reservoir cap **38.** (Although not shown here, the wider fill-side of the reservoir will be sealed following completion of the drug loading and formulating processes described herein.) Metal conductors **36** can deliver electric current through reservoir cap **38** at the desired time of opening the reservoir to initiate release of drug formulation **46.** Dielectric layer **32** and top passivation layer **34** are also shown.

In one embodiment, the matrix of a solid form of a drug comprises lyophilized, non-crystalline drug. In one variation, the excipient material is a pharmaceutically-acceptable solvent in which the drug has significant solubility but does not dissolve the pre-existing solid matrix of drug to an extent that interferes with the requirements of dosing for a particular application, and in addition promotes re-dissolution of the drug upon release of the drug/excipient from the reservoir.

The drug storage and delivery device, which includes one or more reservoirs, can take a wide variety of forms. For example, the drug storage and delivery device can comprise a microchip chemical delivery device, a pump (such as an implantable osmotic or mechanical pump), a drug-eluting stent, or a combination thereof.

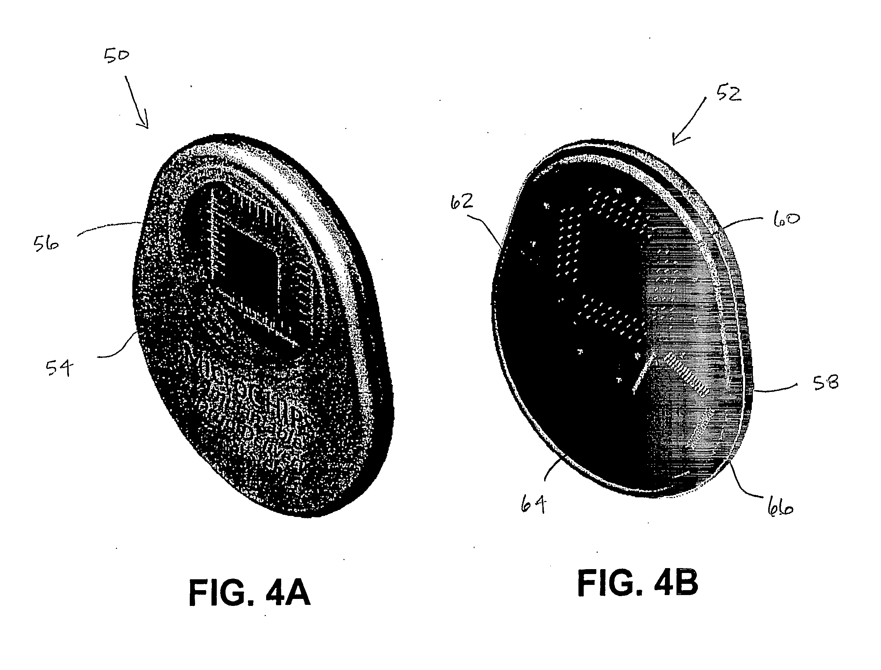

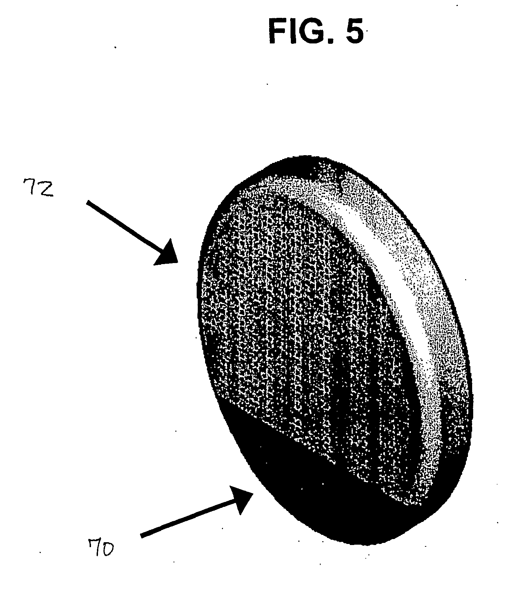

**FIGS. 4A-B** and **FIG. 5** illustrate two possible configurations of implantable drug storage and delivery devices. **FIG. 4A** shows the exterior of device **50** which includes a titanium hermetic enclosure **54.** This figure also shows the release side/surface of the body portion **56** that includes the reservoirs containing the solid drug formulation described herein. **FIG. 4B** shows the interior portion **52** of device **50 ,** which includes ASIC **60,** microprocessor **58,** capacitor **62,** battery **64,** and wireless telemetry antenna **66.** **FIG. 5** shows another embodiment of the device which includes a first portion **72** that includes the reservoirs containing the solid drug formulation described herein, and a second portion **70** that includes all of the control elements (e.g., electronics, power supply, wireless telemetry, etc.)

In preferred embodiments, the device is an implantable device for sustained drug delivery, which comprises one or more reservoirs for containing (storing) the drug formulation until it is released for delivery/administration to the patient. In one embodiment, the formulation of drug matrix with liquid pharmaceutically-acceptable excipient material dispersed throughout pores or interstices within the matrix will be satisfactorily stable over an extended period (e.g., 2, months, 4, months, 6 months, 9 months, 12 months, etc.).

Representative examples of implantable devices that could be adapted for use with the formulations described herein include implantable pumps (e.g., mechanical pumps like those made by Medtronic, MiniMed, and Arrow, or osmotic pumps like DUROS^{™} or Viadur^{™}), stents (vascular or peripheral), and microchip chemical delivery devices (e.g., U.S. Patent No. 5,797,898 to Santini et al., U.S. Patent No. 6,527,762 to Santini et al, U.S. Patent No. 6,656,162 to Santini et al). In other embodiments, the device body with reservoirs can be part of an external system for mixing a drug with a carrier fluid for subsequent delivery, e.g., intravenous delivery, of a solution of drug (e.g., U.S. Patent No. 6,491,666 to Santini et al.). In yet another embodiment, the implantable drug delivery device is a medical stent having microfabricated reservoirs in the body of the stent, e.g., on its exterior surface, its interior surface, or loaded into apertures extending through the body of the stent. Such a stent optionally could include a biodegradable or bioerodible coating to protect the pharmaceutical formulation before and during implantation and/or to delay drug release.

Other methods and multi-reservoir devices for controlled release of drug are described in U.S. Patent Application Publications Nos. 2002/0107470 A1, 2002/0072784 A1, 2002/0138067 A1, 2002/0151776 A1, 2002/0099359 A1, 2002/0187260 A1, and 2003/0010808 A1; PCT WO 2004/022033 A2; PCT WO 2004/026281; and U.S. Patent No. 6,123,861.

### Device for Storage and Exposure of Chemical Sensor

In another aspect, the reservoir filling methods and compositions can be adapted for use in sensor applications. For example, a chemical-based sensor, for example in the form of a gel-bound enzyme, can be loaded into the reservoirs, and then the reservoir can be backfilled with a nonsolvent, such as a PEG, which prevents an air pocket in the reservoir from blocking contact between the chemical based sensor and a physiological fluid (or other environmental component of interest) from outside of the reservoir. See, e.g., U.S. Patent No. 6,551,838 to Santini et al., which describes sensing devices having an array of reservoirs loaded with various chemical sensors for a range of biomedical applications.

### Device Body and Reservoirs

The device comprises a body portion, i.e., a substrate, that includes one or more microreservoirs, each microreservoir containing a microquantity of the drug and the excipient. In various embodiments, the body portion comprises silicon, a metal, a ceramic, a polymer, or a combination thereof. Preferably each reservoir is formed of hermetic materials (e.g., metals, silicon, glasses, ceramics) and is hermetically sealed by a reservoir cap. In various embodiments, the body portion is in the form of a chip, a disk, a tube, a sphere, or a stent.

In a preferred embodiment, the device includes a plurality of the reservoirs located in discrete positions across at least one surface of the body portion.

Microreservoirs can be fabricated in a structural body portion using any suitable fabrication technique known in the art. Representative fabrication techniques include MEMS fabrication processes or other micromachining processes, various drilling techniques (e.g., laser, mechanical, and ultrasonic drilling), and build-up techniques, such as LTCC (low temperature co-fired ceramics). The surface of the microreservoir optionally can be treated or coated to alter one or more properties of the surface. Examples of such properties include hydrophilicity/ hydrophobicity, wetting properties (surface energies, contact angles, etc.), surface roughness, electrical charge, release characteristics, and the like.

As used herein, the term "microreservoir" refers to a concave-shaped solid structure suitable for releasably containing a material, wherein the structure is of a size and shape suitable for filling with a microquantity of the material, which comprises a drug. In one embodiment, the microreservoir has a volume equal to or less than 500 µL (e.g., less than 250 µL, less than 100 µL, less than 50 µL, less than 25 µL, less than 10 µL, etc.) and greater than about 1 nL (e.g., greater than 5 nL, greater than 10 nL, greater than about 25 nL, greater than about 50 nL, greater than about 1 µL, etc.). The shape and dimensions of the microreservoir can be selected to maximize or minimize contact area between the drug material and the surrounding surface of the microreservoir.

As used herein, the term "microquantity" refers to small volumes between 1 nL and 10 µL. In one embodiment, the microquantity is between 1 nL and 1 µL. In another embodiment, the microquantity is between 10 nL and 500 nL.

In other embodiments, the reservoirs are larger than microreservoirs and can contain a quantity of drug formulation larger than a microquantity. For example, the volume of each reservoir can be greater than 10 µL (e.g., at least 20 µL, at least 50 µL, at least 100 µL, at least 250 µL, etc.) and less than 1,000 µL (e.g., less than 900 µL, less than 750 µL, less than 500 µL, less than 300 µL, etc.). These may be referred to as macro-reservoirs and macro-quantities, respectively. Unless explicitly indicated to be limited to either micro- or macro-scale volumes/quantities, the term "reservoir" is intended to include both.

In a preferred embodiment, the device comprises a microchip chemical delivery device. In other embodiments, the device could include polymeric chips or devices composed of non-silicon based materials that might not be referred to as "microchips." In one embodiment, the device could comprise an osmotic pump, for example, the DUROS^{™} osmotic pump technology (Alza Corporation) included in commercial devices such as VIADUR™ (Bayer Healthcare Pharmaceuticals and Alza Corporation).

### Drug or Sensor Material

### Drug

As used herein, the term "drug" is essentially any therapeutic or prophylactic agent, which desirably is provided in a solid form, particularly for purposes of maintaining or extending the stability of the drug over a commercially and medically useful time, e.g., during storage in a drug delivery device until the drug needs to be administered. The solid drug matrix may be in pure form or in the form of solid particles of another material in which the drug is contained or dispersed. As used herein, "pure form" of the drug includes the active pharmaceutical ingredient (API), residual moisture, and any chemical species combined with the API in a specific molar ratio that is isolated with the API during preparation of the API (for instance, a counterion) and which has not been added as an excipient. The drug is in a dry solid matrix form.

The term "dry solid" means a monolithic solid mixture. The terms "pre-form" and "pellet" refers to a small, solid form of the drug matrix loaded with the solidified excipient material.

The drug can comprise small molecules, large (i.e., macro-) molecules, or a combination thereof. In one embodiment, the large molecule drug is a protein or a peptide. In various other embodiments, the drug can be selected from amino acids, vaccines, antiviral agents, gene delivery vectors, interleukin inhibitors, immunomodulators" neurotropic factors, neuroprotective agents, antineoplastic agents, chemotherapeutic agents, polysaccharides, anti-coagulants (e.g., LMWH, pentasaccharides), antibiotics (e.g., immunosuppressants), analgesic agents, and vitamins. In a preferred embodiment, the drug is a protein. Examples of suitable types of proteins include, glycoproteins, enzymes (e.g., proteolytic enzymes), hormones or other analogs (e.g., LHRH, steroids, corticosteroids, growth factors), antibodies (e.g., anti-VEGF antibodies, tumor necrosis factor inhibitors), cytokines (e.g., α-, β-, or γ-interferons), interleukins (e.g., IL-2, IL-10), and diabetes/obesity-related therapeutics (e.g., insulin, exenatide, PYY, GLP-1 and its analogs). In one embodiment, the drug is a gonadotropin-releasing (LH-RH) hormone analog, such as leuprolide. In another exemplary embodiment, the drug comprises parathyroid hormone, such as a human parathyroid hormone or its analogs, e.g., hPTH(1-84) or hPTH(1-34). In a further embodiment, the drug is selected from nucleosides, nucleotides, and analogs and conjugates thereof. In yet another embodiment, the drug comprises a peptide with natriuretic activity, such as atrial natriuretic peptide (ANP), B-type (or brain) natriuretic peptide (BNP), C-type natriuretic peptide (CNP), or dendroaspis natriuretic peptide (DNP).

The methods described herein are particularly useful for drugs that comprise molecules that are unstable in solution, such as aqueous solution. The term "unstable in solution" refers to molecules that may undergo reaction or structural or conformational changes that result in a loss of bioactivity or otherwise render them unsuitable for an intended use. Examples of the types of mechanisms inducing these changes include self-degradation, aggregation, deamidation, oxidation, cleavage, refolding, hydrolysis, conformational changes, and other chemical mechanisms. For example, proteolytic enzymes are known to undergo autolysis. As another example, some proteins form aggregates or undergo deamidation. Non-proteins also may be unstable.

### Sensor Material

In an alternative embodiment, the devices and methods described herein can be used or readily adapted to store and expose a sensor material (particularly one in solid form) in the one or more reservoirs. A wide variety of sensor materials can be used, depending upon the ultimate application. As used herein, the term "sensor material" refers to essentially any reactive chemical species. The reactive chemical species can be a drug compound. In one embodiment, the device for sensing includes multiple discrete reservoirs and optionally includes one or more drugs for release.

In one embodiment, the device comprises a chemical-based sensor which incorporates a gel-bound enzyme at the back (fill side) of a reservoir. The excipient material could be a PEG which prevents an air pocket in the reservoir from blocking the contact between physiological fluid and the chemical based sensor.

In one of the sensor device embodiments, the excipient material comprises or forms a semi-permeable membrane over the sensor material. For example, Nafion can be used as a semi-permeable membrane with glucose oxidase as the sensor material.

### Processing Excipients

In the drying or lyophilization processes, the drug may be processed with one or more additives (i.e., processing excipients). Representative examples of such additives include surfactants, lyoprotectants, and cryoprotectants. Selection of an appropriate additive will depend on the particular drug and drying/lyophilization process to be used. In one embodiment, such additives comprise a pharmaceutically acceptable excipient. The choice and amounts of processing excipient for a particular formulation depend on a variety of factors and can be selected by one skilled in the art. Examples of these factors include the type and amount of drug, the particle size and morphology of the solid form of the drug, the chemical nature or properties of the drug, and the desired properties and route of administration of the final formulation. Examples of types of pharmaceutically acceptable processing excipients include bulking agents, wetting agents, stabilizers, crystal growth inhibitors, antioxidants, antimicrobials, preservatives, buffering agents (e.g., acids, bases), surfactants, desiccants, dispersants, osmotic agents, binders (e.g., starch, gelatin), disintegrants (e.g., celluloses), glidants (e.g., talc), diluents (e.g., lactose, dicalcium phosphate), color agents, lubricants (e.g., magnesium stearate, hydrogenated vegetable oils) and combinations thereof. Other suitable pharmaceutically acceptable processing excipients include most carriers approved for parenteral administration, including water, saline, Ringer's solution, Hank's solution, and solutions of glucose, lactose, dextrose, mannitol, ethanol, glycerol, albumin, and the like. In one embodiment, the processing excipient could include one or more cyclodextrins.

### Void-Displacing Excipient Material

The excipient material is added in liquid form to the solid matrix form of the drug (or sensor material), so that it can impregnate the drug, substantially filling pores, voids, and interstices, and eliminating air bubbles or pockets from the matrix, when contained in a reservoir of a drug storage and delivery device. Once the excipient material is in place (e.g., has impregnated the pores of the solid drug matrix), then the liquid form excipient material can either remain in liquid form or be converted to a solid or semi-solid form. The excipient material preferably enhances handling, stability, solubility, and dispersibility of the drug or sensor material.

The term "excipient material" refers to any non-active ingredient of the formulation intended to facilitate delivery and administration by the intended route. It preferably is pharmaceutically acceptable, which means that it is an ingredient in the dosage form other than the active ingredient that, in the quantities required for the device, will not prevent marketing approval for therapeutic human use by world wide regulatory agencies.

The excipient material is a non-solvent for the drug. As used herein, the term "nonsolvent" refers to a solvent in which the drug solubility is sufficiently low that less than 10% of the drug-containing matrix will dissolve in the solvent in the reservoir over the useful lifetime of the storage and release device for the drug.

In various embodiments, at least one of the one or more excipient materials is a solid, a liquid, a semi-solid, or a gel, at ambient conditions. As used here, "ambient conditions" are about 20 °C and atmospheric pressure.

In one embodiment, the excipient material comprises a compound that interacts (e.g., on a molecular level) with the drug molecule in a selected, desirable manner, for example to enhance storage or administration (e.g., by enhancing the solubility) of the drug. Such an excipient material may be known in the art as a "delivery modifier." For example, delivery modifiers are known in the art for use in the oral delivery of parathyroid hormone (PTH). The delivery modifiers may facilitate passage of the drug through lipid layers in tissue.

In one embodiment, the excipient material is non-aqueous. In one embodiment, the non-aqueous excipient material is a pharmaceutically acceptable liquid.

In some embodiments, the excipient material comprises a polymer. In one embodiment, the polymer comprises polyethylene glycol (PEG), e.g., typically one having a molecular weight between about 100 and 10,000 Daltons. In one embodiment, the excipient material includes PEG 200. In another embodiment, the excipient material includes a PEG that is solid at body temperature, e.g., between about 35 and 40 °C. In one embodiment, a PEG that is a solid at body temperature and a liquid at a temperature slightly above body temperature is used (e.g. PEG 1450). Other polymers, such as poly lactic acid (PLA), poly glycolic acid (PGA), copolymers thereof (PLGA), or ethyl-vinyl acetate (EVA) polymers. In other embodiments, the excipient material could be a pharmaceutically acceptable oil (e.g., sesame oil).

In one embodiment, the excipient material includes a saturated drug solution. That is, the excipient material comprises a liquid solution formed of the drug dissolved in a solvent for the drug. The solution is saturated so that the solvent does not dissolve the solid matrix form of the drug. The saturated solution acts as a non-solvent excipient material, substantially filling pores and voids in the solid matrix.

In another embodiment, the excipient material comprises a pharmaceutically-acceptable perhalohydrocarbon or unsubstituted saturated hydrocarbon. See, for example, U.S. Patent No. U.S. Patent No. 6,264,990 to Knepp et al., which describes anhydrous, aprotic, hydrophobic, non-polar liquids, such as biocompatible perhalohydrocarbons or unsubstituted saturated hydrocarbons, such as perfluorodecalin, perflurobutylamine, perfluorotripropylamine, perfluoro-N-methyldecahydroquindine, perfluoro-octohydro quinolidine, perfluoro-N-cyclohexylpyrilidine, perfluoro-N,N-dimethylcyclohexyl methylamine, perfluoro-dimethyl-adamantane, perfluorotrimethylbicyclo (3.3.1) nonane, bis(perfluorohexyl) ethene, bis(perfluorobutyl) ethene, perfluoro-1-butyl-2-hexyl ethene, tetradecane, methoxyflurane and mineral oil.).

In one embodiment, the pharmaceutically-acceptable excipient material comprises dimethyl sulfoxide (DMSO), glycerol or ethanol.

While it would generally be desirable to use water soluble/miscible pharmaceutically-acceptable excipient materials for use in microchip devices, it is envisioned that such a limitation is not required in all cases or with all reservoir means, for example where there is either a supplemental means of accelerating the release of the drug formulation from a reservoir or if the release is otherwise "non-passive," as with an osmotic pump.

In certain embodiments, the excipient material can be one that would not ordinarily be considered as ingredient in a dosage form. Where the implantable drug delivery device comprises one or more discrete reservoirs of small volume, e.g., microreservoirs, then it may be desirable to use organic solvents that are not possible to use in large amounts, for example due to toxicity concerns. In various embodiments, the solvents listed in Table 1 can be used as the excipient material if the device reservoir volumes are small enough to ensure that the daily exposure to the excipient cannot exceed predetermined limits, for example described in *ICH Guideline Q3C: Impurities: Residual Solvents.*

**TABLE 1: EXCIPIENT MATERIALS AND EXPOSURE LIMITS**

| **Excipient** | **Daily limit (mg)** | **Excipient** | **Daily limit (mg)** |
|---|---|---|---|
| Benzene | 0.02 | 1,1,2-Trichloroethene | 0.8 |
| Carbon tetrachloride | 0.04 | Xylene | 21.7 |
| 1,2-Dichloroethane | 0.05 | Acetic acid | 50 |
| 1,1-Dichloroethene | 0.08 | Acetone | 50 |
| 1,1,1-Trichloroethane | 15 | Anisole | 50 |
| Acetonitrile | 4.1 | 1-Butanol | 50 |
| Chlorobenzene | 3.6 | 2-Butanol | 50 |
| Chloroform | 0.6 | Butyl acetate | 50 |
| Cyclohexane | 38.8 | tert-Butylmethyl ether | 50 |
| 1,2-Dichloroethene | 18.7 | Cumene | 50 |
| Dichloromethane | 6.0 | Dimethyl sulfoxide | 50 |
| 1,2-Dimethoxyethane | 1.0 | Ethanol | 50 |
| N,N-Dimethylacetamide | 10.9 | Ethyl acetate | 50 |
| N,N-Dimethylformamide | 8.8 | Ethyl ether | 50 |
| 1,4-Dioxane | 3.8 | Ethyl formate | 50 |
| 2-Ethoxyethanol | 1.6 | Formic acid | 50 |
| Ethyleneglycol | 6.2 | Heptane | 50 |
| Formamide | 2.2 | Isobutyl acetate | 50 |
| Hexane | 2.9 | Isopropyl acetate | 50 |
| Methanol | 30.0 | Methyl acetate | 50 |
| 2-Methoxyethanol | 0.5 | 3-Methyl-1-butanol | 50 |
| Methylbutyl ketone | 0.5 | Methylethyl ketone | 50 |
| Methylcyclohexane | 11.8 | Methylisobutyl ketone | 50 |
| N-Methylpyrrolidone | 5.3 | 2-Methyl-1-propanol | 50 |
| Nitromethane | 0.5 | Pentane | 50 |
| Pyridine | 2.0 | 1-Pentanol | 50 |
| Sulfolane | 1.6 | 1-Propanol | 50 |
| Tetrahydrofuran | 7.2 | 2-Propanol | 50 |
| Tetralin | 1.0 | Propyl acetate | 50 |
| Toluene | 8.9 | | |

### II. Methods for Making the Formulation

In one embodiment, a method is provided for making a drug formulation which comprises (a) providing a drug in dry, porous matrix form; and (b) adding to the drug matrix (i.e., "backfilling") a liquid pharmaceutically-acceptable excipient material which sufficiently fills the pores and interstices within the matrix that it promotes redissolution of the drug upon administration. The excipient may solidify or remain liquid depending on the administration requirements. By filling the pores and interstices with the liquid pharmaceutically-acceptable excipient material, the air (or other gas) advantageously is displaced, as the presence of the gas could otherwise inhibit re-dissolution of the drug upon administration (e.g., upon exposure of the drug formulation to physiological fluids). The excipient material may also enhance the stability as well as the redissolution of the drug upon release into the physiological medium by effectively lowering the local concentration of the drug upon dissolution to a concentration in the physiological medium that is not saturated; in the absence of the excipient material, the dry formulated drug may, upon dissolution, exceed saturation and precipitate, denature, and/or aggregate. This formulation can be made, stored, and used in a variety of devices and drug delivery systems.

### III. Methods for Loading Device Reservoirs with the Drug Formulation

A variety of methods can be used for loading a drug storage and delivery device with a drug formulation that includes a solid form of a drug. In a first technique, the drug is fluidized, either by dissolving or dispersing the solid drug in a volatile liquid medium or by heating to form a molten drug formulation. The fluidized drug is then introduced into the reservoirs and transformed (e.g., by removing the volatile liquid medium or cooling the molten material), at least partially, into a solid drug form. In the second technique, the solid drug formulation is formed into a suitable pellet that is then loaded into the reservoirs.

### Making Drug Formulation Directly in Delivery Device Reservoir

### Methods Using Volatile Liquid Medium

In one embodiment, the method comprises (a) providing a liquid which comprises a drug dissolved or dispersed in a volatile liquid medium; (b) depositing a quantity of the liquid into at least one reservoir of a drug storage and delivery device; (c) drying the quantity by volatilizing the volatile liquid medium to produce a dry, porous matrix of the drug inside at least one reservoir; and (d) adding to the drug matrix a liquid excipient material which fills or substantially fills the pores and interstices within the matrix. Preferably, the liquid excipient material fills all or substantially all of the space within at least one reservoir not otherwise occupied by the drug matrix. One embodiment of this method is shown in **FIG. 2****.** Empty reservoir **31** is provided and first filled with a drug solution **40** (or suspension, etc.). The solution is dried (or lyophilized, etc.) to yield a solid, porous drug matrix **42.** Then, a fluidized excipient material **44** is added into the matrix to yield drug formulation **46** which is a drug matrix with infiltrated excipient.

### Step (a)

The drug can be combined with a suitable volatile liquid medium to form a solution or suspension or emulsion of the drug, using techniques known in the art. In one embodiment, the volatile liquid medium comprises a solvent for the drug so that the liquid vehicle comprises a solution of the active agent dissolved in the solvent. In another embodiment, the volatile liquid medium comprises a non-solvent for the drug so that the liquid vehicle comprises a suspension or emulsion of the active agent dispersed in the non-solvent.

As used herein, the "volatile liquid medium" refers to a liquid vehicle in which the drug is provided before/for undergoing lyophilization or drying. It may be a solvent or a non-solvent for the drug, and it can be volatilized (e.g., by evaporation or sublimation or a combination thereof) to leave the dissolved or suspended drug. The selection of the volatile liquid medium depends, at least in part, on the chosen drug and the desired conditions of lyophilization or drying (e.g., temperature, pressure, speed of volatilization, etc.). The volatile liquid medium preferably is selected to minimize its reaction with the drug and to avoid promoting degradation of the drug before the liquid medium can be volatilized.

The volatile liquid medium may be aqueous or non-aqueous. Representative examples of aqueous volatile liquid media include water, saline, Ringer's solution, Hank's solution, and aqueous solutions of glucose, lactose, dextrose, mannitol, ethanol, glycerol, albumin, and the like.

The volatile liquid medium may include one or more additives, such as those described above. Examples of these additives include surfactants and other excipient materials. In one embodiment for preparing a stable protein formulation from a protein sensitive to air-liquid interfaces, the additive comprises a polyoxyethylene sorbitan fatty acid ester, particularly polyoxyethylene sorbitan monooleate (i.e., TWEEN^{™} 80, polysorbate 80). See Ha, et al., J. Pharma. Sci., 91(10):2252-64 (2002).

In certain embodiments, the drug delivery device includes small reservoir volumes. Because of the small reservoir volume, many volatile liquids may be used that ordinarily would not be considered during production of a dosage form. If the daily exposure to residual liquid in the finished dosage form will not exceed the limits in the Table 1 (Reference: ICH Guideline Q3C: Impurities: Residual Solvents), then the listed volatile excipients could be used during production of a dosage form if required.

### Step (b)

The solution or suspension of drug in the volatile liquid medium can be deposited into the reservoir by a variety of techniques, such as microinjection or other techniques known in the art.

### Step (c)

The term "drying" refers to removal of the volatile liquid medium by evaporation, sublimation, or a combination thereof. In one embodiment, the quantity of liquid is frozen after the deposition of step (b) and before the drying of step (c). Optionally, the drying of step (c) can include reheating the frozen quantity, subjecting the quantity of liquid to a sub-atmospheric pressure, or both.

The drying and lyophilization processes are, or are adapted from, standard bulk processing techniques in the art. A typical lyophilizer consists of a chamber for vacuum drying, a vacuum source, a freezing mechanism, a heat source, and a vapor removal system. For some drugs, the vacuum pressure in the lyophilization process is as low as 0.1 mm Hg. In one embodiment, microscale drying and/or lyophilization methods and equipment as described in U.S. Patent Application Publication No. 2004/0043042 A1 are used.

### Step (d)

Following drying, a liquid excipient material is added to the drug matrix which fills or substantially fills the pores and interstices within the matrix. In one embodiment of this method, after the step of depositing the liquid on the dry solid, the penetration of the voids in the solid by the liquid may be facilitated by a number of techniques. Examples of the techniques include pulling sufficient vacuum to accomplish the penetration, adding sufficient heat to the system to accomplish the penetration by lowering the viscosity of the liquid, or a combination of these techniques. In addition, the same liquid, or a different liquid, can be used to occupy volume, if any, in the reservoir that was not filled with the drug matrix and the first filling fluid if gas remaining in the region inhibited re-dissolution or release of the drug.

### Molten Fill

In one embodiment, the drug is dispersed or dissolved in molten excipient material during device filling, as in hot melt extrusion. The standard practice of hot melt extrusion involves temperatures exceeding 100 °C. In one embodiment, heat sensitive drugs are mixed with an excipient material that is held above the melting point of the solution mixture until reservoir filling is complete, where the storage and expected use temperatures are below the melting point. In a preferred embodiment, a polyethylene glycol (PEG) is used as the excipient material, and the hot melt extrusion is carried out at relatively low (<60 °C) temperatures that are acceptable for many peptide and protein drugs.

### Transferring Preformed Drug into Delivery Device Reservoir

In another embodiment, the solid drug formulation is formed in a recess of a substrate (i.e., a mold), or discrete reservoirs, to produce an individual pre-form (i.e., pellets or cakes). This pre-form retains the shape of the mold recess, and it can be transferred into a reservoir in a drug storage and delivery device, e.g., an implantable pump or other implantable drug delivery device. Alternatively, the pre-form (or more likely multiple pre-forms) can be transferred into a container (e.g., a glass vial) for long term storage and later used with standard (simple) delivery systems (e.g., a syringe).

In one embodiment, a binder is added to the pre-form to give it sufficient structural integrity to be cast and handled without damage. For example, the binder could be an excipient material added in liquid form to the solid drug matrix in the mold, which transforms from liquid to solid or semi-solid after infiltrating the drug matrix. In preferred embodiments, the binder is a polymer, such as a low molecular weight PEG. For example, the process could include heating the binder to its melting point, injecting it onto a drug pre-form, allowing it to infiltrate the perform with slight heating under vacuum, and then allowing the binder to cool to room temperature and solidify. The resulting solid pre-form comprises lyophilized drug particles encapsulated by solid excipient material.

In one embodiment, a drug formulation is made in the form of pellets (i.e., pre-forms) obtained by (a) providing a liquid which comprises a drug dissolved or dispersed in a volatile liquid medium; (b) depositing a quantity of the liquid into at least one reservoir; (c) drying the quantity by volatilizing the volatile liquid medium to produce a dry, porous matrix of the drug inside at least one reservoir; (d) adding to the drug matrix a liquid excipient material which fills the pores and interstices within the matrix; (e) solidifying the liquid pharmaceutically-acceptable excipient material to form a pellet of drug and excipient; and (f) removing the pellet from the at least one reservoir.

Bulk quantities of the drug formulation can be made, for example, by carrying out the process in a plurality of reservoirs, in series or simultaneously, to form a plurality of pellets of the drug formulation. The plurality of pellets can be combined and loaded into a vial or other container for stable storage of the drug. The vial or other container preferably is adapted to facilitate reconstitution (e.g., by dissolution in a pharmaceutically acceptable liquid or dispersion in a pharmaceutically acceptable liquid or gas) and administration of the drug formulation (e.g., by oral administration or by injection, pulmonary, or other parenteral administration routes).

The reservoirs-particularly microreservoirs-loaded with transferred pellets may be "topped off' with the same or a different excipient material in order to eliminate (i.e., displace) any gas pockets that could lead to bubbles in the reservoir, as such bubbles could interfere with release/dissolution of the drug formulation. Eliminating bubbles may be particularly critical for microreservoirs or other reservoirs having small or micron size openings for drug release.

The invention can be further understood with reference to the following non-limiting examples.

### Examples

The release performance of different formulations of leuprolide, a potent leutenizing hormone-releasing hormone (LHRH) analog, from a microchip drug delivery device was assessed. The formulations that were considered included solution phase forms, a lyophilized form which included a dissolution promoting excipient, and a lyophilized form which did not include any additional material. Releases of the different drug forms from the reservoirs of the device were carried out using reservoir opening by electro-resistive ablation. The releases were performed using a flow cell apparatus. Following a release activation, a mobile phase (aqueous phosphate buffered saline solution) was flowed through the cell at periodic intervals. Individual effluent fractions were collected and the quantities of leuprolide released and recovered in each fraction were determined by HPLC analysis using a method specific for the leuprolide monomer.

### Example 1: Release of Lyophilized Leuprolide from Microreservoirs With Secondary Fill of PEG 1450

### Loading Microchip with Drug Solution

Reservoirs of a microchip were filled with an aqueous solution of the drug. The solution was prepared by dissolving leuprolide acetate, as received from the commercial vendor, in water. No other materials were added to the solution. The leuprolide concentration, expressed as the equivalent leuprolide free base concentration, was 190 mg/mL. Each reservoir was filled with 100 nL of solution.

### On-Chip Lyophilization

Immediately following the filling the chip, the chip and its contents were frozen, and the chip was transferred to the pre-chilled shelf of a lyophilizer (-40 °C). The aqueous solvent was sublimated under reduced pressure (lyophilization). The lyophilization appeared successful, as no melt-back was observed and the lyophilized cakes retained their shape and volume upon pressure equilibration.

### Addition of Dissolution Promoting Excipient

Polyethylene glycol with a nominal molecular weight of 1450 g/mole (PEG 1450, melting point approximately 42 °C) was heated above its melting point and dispensed onto the lyophilized cakes of leuprolide. The volume of PEG 1450 dispensed onto each cake was 100 nL. Rapid uptake of PEG 1450 by the cake was observed. The chip, containing lyophilized leuprolide and PEG 1450, was placed in a vacuum chamber at approximately 50 °C and for approximately 1 hour to promote outgassing of trapped gas (air) within the leuprolide-PEG 1450 matrix.

### Measuring Release of Drug

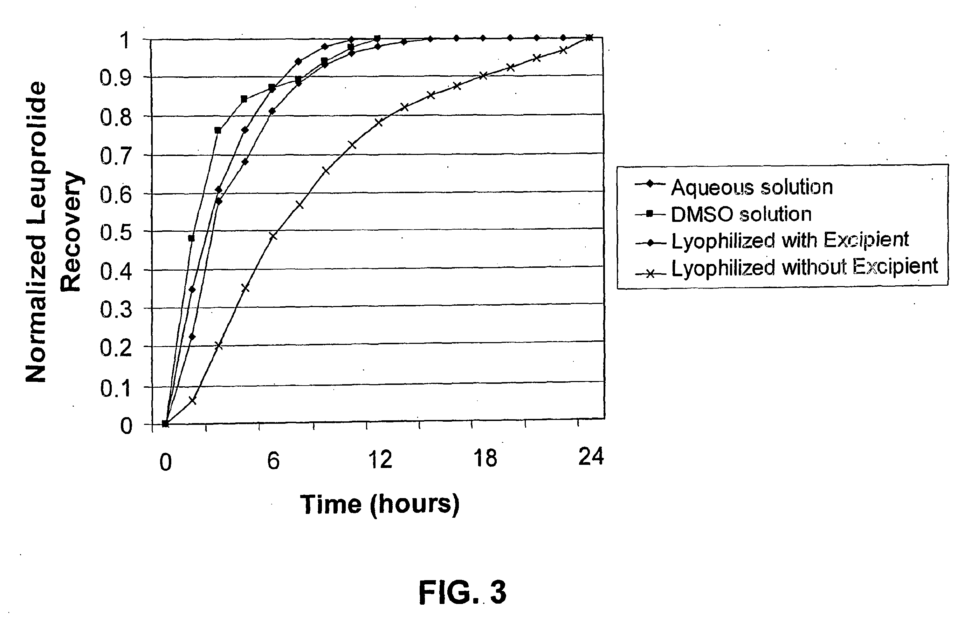

The reservoirs of the chip, containing the solid-solid dispersion of leuprolide in PEG 1450, were sealed using an adhesive foil. The sealed chip was packaged in a flow cell, and releases were activated at 24 hour intervals. At 90-minute intervals a volume of mobile phase was passed through the flow cell and assayed for leuprolide content using a reverse phase HPLC method specific for leuprolide monomer. Leuprolide was detected in the effluent stream. Reproducible release kinetics and mass recoveries were observed, with mass recoveries typically exceeding 90 % of the theoretical yield. A representative release profile is presented in **FIG. 3****.**

### Example 2: Release of Lyophilized Leuprolide Without Secondary Fill - Prior Art

### Loading Microchip with Drug Solution

Reservoirs of a microchip were filled with an aqueous solution of the drug. The solution was prepared by dissolving leuprolide acetate, as received from the commercial vendor, in water. No other materials were added to the solution. The leuprolide concentration, expressed as the equivalent leuprolide free base concentrations, was 180 mg/mL. Each reservoir was filled with 100 nL of solution.

### On-Chip Lyophilization

Immediately following the filling of the chip, the chip and its contents were frozen, and the chip was transferred to the pre-chilled shelf of a lyophilizer (-40 °C). The aqueous solvent was sublimated under reduced pressure (lyophilization). The lyophilization appeared successful, as no melt-back was observed and the lyophilized cakes retained their shape and volume upon pressure equilibration.

### Measuring Release of Drug

The reservoirs of the chip, containing dry lyophilizate, were sealed using an adhesive foil. The sealed chip was packaged in a flow cell, and releases were activated in 24 hour intervals. At 90-minute intervals a volume of mobile phase was passed through the flow cell and assayed for leuprolide content using a reverse phase HPLC method specific for leuprolide monomer. Leuprolide was detected in effluent fractions. Variable release kinetics and mass recoveries were observed. Compared to the releases of the lyophilized leuprolide for which the void volume of the lyophilized cake had been displaced with PEG 1450, release kinetics were uniformly slower and mass recoveries were lower. A representative release profile for the dry, lyophilized leuprolide is presented in FIG. 3.

### Example 3: Release of Solution Phase Leuprolide; Leuprolide in DMSO

As a basis for comparing the release properties of lyophilized leuprolide formulations, releases were performed from chips containing solution phase leuprolide.

### Loading Microchip with Drug Solution

Reservoirs of a microchip were filled with a solution of the drug in dimethyl sulfoxide (DMSO). The solution contained leuprolide acetate, as received from the commercial vendor, and DMSO. No other materials were added to the solution. The leuprolide concentration, expressed as the equivalent leuprolide free base concentration, was 170 mg/mL. Each reservoir was filled with 100 nL of solution.

### Measuring Release of Drug

The reservoirs of the chip, containing solution phase leuprolide in DMSO, were sealed using an adhesive foil. The sealed chip was packaged in a flow cell, and releases were activated in 24 hour intervals. At 90-minute intervals a volume of mobile phase was passed through the flow cell and assayed for leuprolide content using a reverse phase HPLC method specific for leuprolide monomer. Leuprolide was detected in effluent fractions. Reproducible release kinetics and mass recoveries were observed, with mass recoveries typically exceeding 80 % of the theoretical yield. A representative release profile is presented in FIG. 3.

### Example 4: Release of Solution Phase Leuprolide; Leuprolide in Water

As a basis for comparing the release properties of lyophilized leuprolide formulations, releases were performed from chips containing solution phase leuprolide.

### Loading Microchip with Drug Solution

Reservoirs of a microchip were filled with a solution of the drug in water. The solution contained leuprolide acetate, as received from the commercial vendor, and water. No other materials were added to the solution. The leuprolide concentration, expressed as the equivalent leuprolide free base concentration, was 200 mg/mL. Each reservoir was filled with 100 nL of solution.

### Measuring Release of Drug

The reservoirs of the chip, containing aqueous leuprolide, were sealed using an adhesive foil. The sealed chip was packaged in a flow cell, and releases were activated in 24 hour intervals. At 90-minute intervals a volume of mobile phase was passed through the flow cell and assayed for leuprolide content using a reverse phase HPLC method specific for leuprolide monomer. Leuprolide was detected in effluent fractions. Reproducible release kinetics and mass recoveries were observed, with mass recoveries typically exceeding 85 % of the theoretical yield. A representative release profile is shown in FIG. 3.

Table 2 below shows a comparison of the release properties for leuprolide formulations, including lyophilized forms with and without the addition of a dissolution promoting excipient.

**Table 2: Leuprolide Formulation Release Characteristics**

| Formulation | Recovery (after 12 hr), expressed as percent of theoretical fill | Time to 50% of cumulative recovery (after 12 hr) |
|---|---|---|
| Aqueous solution phase | 89 % | 2.8 hr |
| DMSO solution phase | 84% | 1.1 hr |
| Lyophilizate, no secondary fill | 37 % | 4.3 hr |
| Lyophilizate, secondary fill with PEG 1450 | 94% | 2.1 hr |

**FIG. 3** illustrates representative release profiles for solution and solid forms of leuprolide. Reproducible release kinetics and yields are found for the solution phase formulations and for the lyophilized leuprolide in a matrix of PEG 1450. The release kinetics obtained for the lyophilized leuprolide alone are typically variable and slow. It was demonstrated that the use of a solid excipient material could be used to enhance drug release kinetics essentially as well as a liquid excipient material. However, it is believed that, at least for some drugs such as proteins, the solid excipient material may offer greater long term stability of the drug compared to the liquid excipient material, particularly aqueous excipient materials.

## Claims

1. A device for the storage and controlled release of a solid form of a drug comprising:
a body portion;
a plurality of reservoirs located in and defined by the body portion;
a drug in a porous solid monolithic matrix form contained in each of the reservoirs; and
one or more excipient materials dispersed throughout the pores of the drug matrix and substantially filling the space not otherwise occupied by the drug matrix within each of the reservoirs,
wherein the excipient material enhances release of the drug from each reservoir.

2. The device of claim 1, wherein at least one of the one or more excipient materials is solid at ambient conditions.

3. The device of claim 1, wherein at least one of the one or more excipient materials is liquid at ambient conditions.

4. The device of claim 1, wherein at least one of the one or more excipient materials is a semi-solid or gel at ambient conditions.

5. The device of claim 1, wherein the one or more excipient materials are non-aqueous.

6. The device of claim 1, wherein at least one of the one or more excipient materials comprises a polymer.

7. The device of claim 6, wherein the polymer comprises polyethylene glycol.

8. The device of claim 7, wherein the polyethylene glycol has a molecular weight between about 100 and 10,000 Da.

9. The device of claim 1, wherein at least one of the one or more excipient materials comprises a perhalohydrocarbon or unsubstituted saturated hydrocarbon.

10. The device of claim 1, wherein at least one of the one or more excipient materials comprises dimethyl sulfoxide or ethanol.

11. The device of claim 1, wherein at least one of the one or more excipient materials comprises a pharmaceutically-acceptable oil.

12. The device of any one of claims 1 to 11, wherein the drug comprises an amino acid, a peptide, or a protein.

13. The device of any one of claims 1 to 11, wherein the drug is selected from the group consisting of glycoproteins, enzymes, hormones, interferons, interleukins, and antibodies.

14. The device of any one of claims 1 to 11, wherein the drug comprises a human parathyroid hormone, a leutenizing hormone-releasing hormone, a gonadotropin-releasing hormone, or an analog thereof.

15. The device of any one of claims 1 to 11, wherein the drug comprises a natriuretic peptide.

16. The device of any one of claims 1 to 15, wherein the reservoirs are microreservoirs.

17. The device of claim 16, wherein the volume of each reservoir is between 10 nL and 500 nL.

18. The device of claim 1, wherein the reservoirs individually have a volume between 10 µL and 500 µL.

19. The device of any one of claims 1 to 18, wherein the body portion is in the form of a chip, a disk, a tube, a stent, or a sphere.

20. The device of any one of claims 1 to 19, wherein the body portion comprises silicon, a metal, a polymer, a ceramic, or a combination thereof.

21. The device of any one of claims 1 to 20, wherein each reservoir has an opening covered by an impermeable reservoir cap which is selectively ruptured to initiate release of the drug from the reservoir.

22. The device of claim 21, wherein the reservoir cap comprises a metal film which is disintegrated by electrothermal ablation.

23. The device of claim 1, wherein a first excipient material is dispersed throughout pores or interstices within the solid matrix and a second excipient material occupies reservoir space not occupied by the first excipient material or the solid matrix, within each of the reservoirs.

24. The device of claim 1, wherein the one or more excipient materials, upon exposure to an environmental solvent for the drug, promote dissolution of the drug to enhance release of the drug from the reservoir.

25. The device of claim 1, wherein the one or more excipient materials prevent aggregation or precipitation of the drug upon exposure to an environmental fluid to enhance release of the drug from the reservoir.

26. The device of any one of claims 1 to 25, adapted for implantation into a patient.

27. The device of claim 26, wherein the device releases *in vivo* an organic solvent excipient in an amount that is less than the predetermined maximum daily exposure for the organic solvent.

28. A method for making a device for the storage and controlled release of a solid form of a drug comprising:
providing a device body portion having a plurality of reservoirs located therein, the device being adapted for the storage and controlled release of a drug;
providing in the reservoirs a drug in porous solid monolithic matrix form; and
combining with the drug matrix at least one excipient material which substantially fills the pores of the drug matrix to form a drug/excipient composite,
wherein the drug/excipient composite, alone or in combination with another excipient material, substantially fills each of the reservoirs.

29. The method of claim 28, wherein the drug matrix is first provided in the one or more reservoirs and then fluidized excipient material is added to the reservoirs.

30. The method of claim 28, wherein the drug matrix is formed by a method comprising:
dissolving or dispersing a drug in a volatile liquid medium to form a first fluid;
depositing a quantity of the first fluid into each of the reservoirs; and
drying the quantity by volatilizing the volatile liquid medium to produce the solid monolithic matrix of the drug in the reservoirs.

31. The method of claim 28, wherein the at least one excipient material is in a molten state when combined with the drug matrix.

32. The method of claim 28, wherein the drug matrix and the at least one excipient material first are combined together outside of the reservoirs to form a drug/excipient composite and then the drug/excipient composite is loaded into the reservoirs.

33. The method of claim 32, wherein the drug/excipient composite is solidified into a pre-form before being loaded into the reservoirs, each pre-form being shaped to fit into and substantially fill one of the reservoirs.

34. The method of claim 32, wherein the drug/excipient composite is melt-extruded into the reservoirs.

35. The method of claim 29, further comprising solidifying the fluidized excipient material.

36. The method of claim 28, wherein the excipient material comprises a saturated solution of the drug.

37. The method of any one of claims 28 to 36, wherein the reservoirs are microreservoirs.

## Patentansprüche

1. Vorrichtung für die Speicherung und gesteuerte Freisetzung einer festen Form eines Arzneistoffs, umfassend:
einen Körperbereich;
eine Mehrzahl von Reservoirs, platziert in und definiert durch den Körperbereich;
einen Arzneistoff in einer porösen, festen, monolithischen Matrixform enthalten in jedem der Reservoirs; und
ein oder mehrere Hilfsstoffmaterialien, die durch die Poren der Arzneistoffmatrix dispergiert sind, und im Wesentlichen den Raum ausfüllen, der nicht anderweitig besetzt ist durch die Arzneistoffmatrix innerhalb jedes Reservoirs,
wobei das Hilfsstoffmaterial die Freisetzung des Arzneistoffs aus jedem Reservoir verbessert.

2. Vorrichtung gemäß Anspruch 1, wobei zumindest eines der einen oder mehreren Hilfsstoffmaterialien fest ist unter Umgebungsbedingungen.

3. Vorrichtung gemäß Anspruch 1, wobei zumindest eines der einen oder mehreren Hilfsstoffmaterialien flüssig ist bei Umgebungsbedingungen.

4. Vorrichtung gemäß Anspruch 1, wobei zumindest eines der einen oder mehreren Hilfsstoffmaterialien halbfest oder gelartig ist bei Umgebungsbedingungen.

5. Vorrichtung gemäß Anspruch 1, wobei die einen oder mehreren Hilfsstoffmaterialien nicht wässrig sind.

6. Vorrichtung gemäß Anspruch 1, wobei zumindest eines der einen oder mehreren Hilfsstoffmaterialien ein Polymer umfasst.

7. Vorrichtung gemäß Anspruch 6, wobei das Polymer Polyethylenglykol umfasst.

8. Vorrichtung gemäß Anspruch 7, wobei das Polyethylenglykol ein Molekulargewicht zwischen ungefähr 100 und 10.000 Da hat.

9. Vorrichtung gemäß Anspruch 1, wobei zumindest eines der einen oder mehreren Hilfsstoffmaterialien ein Perhalogenkohlenwasserstoff oder einen unsubstituierten gesättigten Kohlenwasserstoff umfasst.

10. Vorrichtung gemäß Anspruch 1, wobei zumindest eines der einen oder mehreren Hilfsstoffmaterialien Dimethylsulfoxid oder Ethanol umfasst.

11. Vorrichtung gemäß Anspruch 1, wobei zumindest eines der einen oder mehreren Hilfsstoffmaterialien ein pharmazeutisch verträgliches Öl umfasst.

12. Vorrichtung gemäß einem der Ansprüche 1 bis 11, wobei der Arzneistoff eine Aminosäure, ein Peptid oder ein Protein umfasst.

13. Vorrichtung gemäß einem der Ansprüche 1 bis 11, wobei der Arzneistoff ausgewählt ist aus der Gruppe bestehend aus Glykoproteinen, Enzymen, Hormonen, Interferonen, Interleukinen und Antikörpern.

14. Vorrichtung gemäß einem der Ansprüche 1 bis 11, wobei der Arzneistoff ein menschliches Nebenschilddrüsenhormon, ein luteinisierendes-Hormon-freisetzendes Hormon, ein Gonadotropin-freisetzendes Hormon oder ein Analogon daraus umfasst.

15. Vorrichtung gemäß einem der Ansprüche 1 bis 11, wobei der Arzneistoff ein natriuretisches Peptid umfasst.

16. Vorrichtung gemäß einem der Ansprüche 1 bis 15, wobei die Reservoirs Mikroreservoirs sind.

17. Vorrichtung gemäß Anspruch 16, wobei das Volumen von jedem der Reservoirs zwischen 10 nL und 500 nL beträgt.

18. Vorrichtung gemäß Anspruch 1, wobei die einzelnen Reservoirs ein Volumen zwischen 10 µL und 500 µL haben.

19. Vorrichtung gemäß einem der Ansprüche 1 bis 18, wobei der Körperbereich die Form eines Plättchens, einer Scheibe, einer Röhre, eines Stents oder einer Kugel hat.

20. Vorrichtung gemäß einem der Ansprüche 1 bis 19, wobei der Körperbereich Silizium, ein Metall, ein Polymer, eine Keramik oder eine Kombination daraus umfasst.

21. Vorrichtung gemäß einem der Ansprüche 1 bis 20, wobei jedes Reservoir eine Öffnung aufweist, die durch eine undurchlässige Reservoirkappe bedeckt ist, welche selektiv durchtrennt ist, um die Freisetzung des Arzneistoffs aus dem Reservoir zu initiieren.

22. Vorrichtung gemäß Anspruch 21, wobei die Reservoirkappe einen Metallfilm umfasst, der durch elektrothermische Ablation zersetzt ist.

23. Vorrichtung gemäß Anspruch 1, wobei ein erstes Hilfsstoffmaterial durch die Poren oder Zwischenräume in der festen Matrix dispergiert ist und ein zweites Hilfsstoffmaterial den Reservoirraum einnimmt, der nicht durch das erste Hilfsstoffmaterial oder die feste Matrix eingenommen wird, innerhalb jedes der Reservoirs.

24. Vorrichtung gemäß Anspruch 1, wobei die einen oder mehreren Hilfsstoffmaterialien, bei Aussetzen einem umgebenden Lösungsmittel des Arzneistoffs, die Auflösung des Arzneistoffs fördern, um die Freisetzung des Arzneistoffs aus dem Reservoir zu verbessern.

25. Vorrichtung gemäß Anspruch 1, wobei die einen oder mehreren Hilfsstoffmaterialien die Aggregation oder Precipitation des Arzneistoffs bei Aussetzen einer umgebenden Flüssigkeit zu verhindern, um das Freisetzen des Arzneistoffs aus dem Reservoir zu verbessern.

26. Vorrichtung gemäß einem der Ansprüche 1 bis 25, angepasst zur Implantation in einen Patienten.

27. Vorrichtung gemäß Anspruch 26, wobei die Vorrichtung in vivo einen organischen Lösungsmittelhilfsstoff freisetzt in einer Menge, die geringer ist als die vorbestimmte maximale tägliche Dosis für das organische Lösungsmittel.

28. Verfahren zum Herstellen einer Vorrichtung zur Speicherung und gesteuerten Freisetzung einer festen Form eines Arzneistoffes, umfassend:
Bereitstellen eines Vorrichtungs-Körperbereichs mit einer Mehrzahl von Reservoirs darin platziert, wobei die Vorrichtung ausgelegt ist für das Speichern und die gesteuerte Freisetzung eines Arzneistoffes;
Bereitstellen, in den Reservoirs, eines Arzneistoffs in poröser fester monolithischer Matrixform; und
Verbinden mindestens eines Hilfsstoffmaterials mit der Arzneistoffmatrix, das im Wesentlichen die Poren der Arzneistoffmatrix füllt, um eine Arzneistoff/Hilfsstoffzusammensetzung zu bilden,
wobei die Arzneistoff/Hilfsstoffzusammensetzung, allein oder in Kombination mit einem weiteren Hilfsstoffmaterial, jedes der Reservoirs im Wesentlichen füllt.

29. Verfahren nach Anspruch 28, wobei die Arzneistoffmatrix zuerst in einer oder mehren Reservoirs bereitgestellt wird und dann verflüssigtes Hilfsstoffmaterial in die Reservoirs hinzugefügt wird.

30. Verfahren nach Anspruch 28, wobei die Arzneistoffmatrix gebildet wird durch ein Verfahren umfassend:
Auflösen oder Dispergieren eines Arzneistoffs in einem flüchtigen flüssigen Medium, um eine erste Flüssigkeit zu bilden;
Aufbringen einer Menge der ersten Flüssigkeit in jedes der Reservoirs; und
Trocknen der Menge durch Verflüchtigen des flüchtigen flüssigen Mediums, um die feste monolithische Matrix des Arzneistoffs in den Reservoirs zu produzieren.

31. Verfahren nach Anspruch 28, wobei zumindest ein Hilfsstoffmaterial in einem geschmolzenen Zustand ist, wenn es mit der Arzneistoffmatrix verbunden wird.

32. Verfahren nach Anspruch 28, wobei die Arzneistoffmatrix und zumindest ein Hilfsstoffmaterial zuerst miteinander verbunden werden außerhalb der Reservoirs, um ein Arzneistoff-/Hilfsstoffzusammensetzung zu bilden und dann die Arzneistoff/Hilfsstoffzusammensetzung in die Reservoirs gefüllt wird.

33. Verfahren nach Anspruch 32, wobei die Arzneistoff/Hilfsstoffzusammensetzung verfestigt wird in einer Vorform bevor sie es in die Reservoirs gefüllt wird, wobei jede Vorform geformt ist, um in eines der Reservoirs zu passen und dieses im Wesentlichen auszufüllen.

34. Verfahren nach Anspruch 32, wobei die Arzneistoff/Hilfsstoffzusammensetzung schmelzextrudiert wird in die Reservoirs.

35. Verfahren nach Anspruch 29, weiter umfassend Verfestigen des verflüssigten Hilfsstoffmaterials.

36. Verfahren nach Anspruch 28, wobei das Hilfsstoffmaterial eine gesättigte Lösung eines Arzneistoffs umfasst.

37. Verfahren nach einem der Ansprüche 28 bis 36, wobei die Reservoirs Mikroreservoirs sind.

## Revendications

1. Dispositif pour le stockage et le dégagement contrôlé d'un médicament sous forme solide, comprenant :
une portion de corps ;
une pluralité de réservoirs situés dans et définis par la portion de corps ;
un médicament sous forme d'une matrice monolithique solide poreuse contenue dans chacun des réservoirs ; et
un ou plusieurs matériaux excipients dispersés dans tous les pores de la matrice du médicament et remplissant sensiblement l'espace qui n'est pas par ailleurs occupé par la matrice de médicament à l'intérieur de chacun des réservoirs,
dans lequel le matériau excipient renforce le dégagement du médicament hors de chaque réservoir.

2. Dispositif selon la revendication 1, dans lequel l'un au moins des un ou plusieurs matériaux excipients est solide dans les conditions ambiantes.

3. Dispositif selon la revendication 1, dans lequel l'un au moins des un ou plusieurs matériaux excipients est liquide dans les conditions ambiantes.

4. Dispositif selon la revendication 1, dans lequel l'un au moins des un ou plusieurs matériaux excipients est un semi-solide ou un gel dans les conditions ambiantes.

5. Dispositif selon la revendication 1, dans lequel les un ou plusieurs matériaux excipients sont non-aqueux.

6. Dispositif selon la revendication 1, dans lequel l'un au moins des un ou plusieurs matériaux excipients comprend un polymère.

7. Dispositif selon la revendication 6, dans lequel le polymère comprend du polyéthylène glycol.

8. Dispositif selon la revendication 7, dans lequel le polyéthylène glycol possède un poids moléculaire entre environ 100 et 10 000 Da.

9. Dispositif selon la revendication 1, dans lequel l'a au moins des un ou plusieurs matériaux excipients comprend un perhalohydrocarbure ou un hydrocarbure saturé non substitué.

10. Dispositif selon la revendication 1, dans lequel l'un au moins des un ou plusieurs matériaux excipients comprend du diméthyl sulfoxyde ou de l'éthanol.

11. Dispositif selon la revendication 1, dans lequel au moins un des un ou plusieurs matériaux excipients comprend une huile pharmaceutiquement acceptable.

12. Dispositif selon l'une quelconque des revendications 1 à 11, dans lequel le médicament comprend un acide aminé, un peptide ou une protéine.

13. Dispositif selon l'une quelconque des revendications 1 à 11, dans lequel le médicament est choisi parmi le groupe comprenant glycoprotéines, enzymes, hormones, interférons, interleukines et anticorps.

14. Dispositif selon l'une quelconque des revendications 1 à 11, dans lequel le médicament comprend une hormone parathyroïde humaine, une hormone dégageant une hormone luténisante, une hormone dégageant une gonadotrophine humaine, ou une hormone analogue.

15. Dispositif selon l'une quelconque des revendications 1 à 11, dans lequel le médicament comprend un peptide natriurétique.

16. Dispositif selon l'une quelconque des revendications 1 à 15, dans lequel les réservoirs sont des microréservoirs.

17. Dispositif selon la revendication 16, dans lequel le volume de chaque réservoir est entre 10 nL et 500 nL.

18. Dispositif selon la revendication 1, dans lequel les réservoirs ont individuellement un volume entre 10 µL et 500 µL.

19. Dispositif selon l'une quelconque des revendications 1 à 18, dans lequel la portion de corps a la forme d'une plaquette, d'un disque, d'un tube, d'un stent ou d'une sphère.

20. Dispositif selon l'une quelconque des revendications 1 à 19, dans lequel la portion de corps comprend du silicone, un métal, un polymère, une céramique, ou une combinaison de ceux-ci.

21. Dispositif selon l'une quelconque des revendications 1 à 20, dans lequel chaque réservoir comporte une ouverture couverte par un capuchon imperméable qui est sélectivement rompu pour démarrer un dégagement du médicament hors du réservoir.

22. Dispositif selon la revendication 21, dans lequel le capuchon de réservoir comprend un film de métal qui est désintégré par ablation électrothermique.

23. Dispositif selon la revendication 1, dans lequel un premier matériau excipient est dispersé dans tous les pores ou les interstices à l'intérieur de la matrice solide, et un second matériau excipient occupe dans le réservoir l'espace qui n'est pas occupé par le premier matériau excipient ou la matrice solide, à l'intérieur de chacun des réservoirs.

24. Dispositif selon la revendication 1, dans lequel lesdits un ou plusieurs matériaux excipients, lorsqu'ils sont exposés à un solvant environnemental pour le médicament, favorisent la dissolution du médicament pour renforcer le dégagement du médicament hors du réservoir.

25. Dispositif selon la revendication 1, dans lequel lesdits un ou plusieurs matériaux excipients empêchent une agrégation ou une précipitation du médicament lors de l'exposition à un fluide environnemental pour renforcer le dégagement du médicament hors du réservoir.

26. Dispositif selon l'une quelconque des revendications 1 à 25, adapté pour l'implantation dans un patient.

27. Dispositif selon la revendication 26, dans lequel le dispositif dégage in vivo un excipient solvant organique dans une quantité qui est inférieure à l'exposition quotidienne maximum prédéterminée pour le solvant organique.

28. Procédé pour réaliser un dispositif pour le stockage et le dégagement contrôlé d'un médicament sous forme solide, comprenant :
de fournir une portion formant corps de dispositif ayant une pluralité de réservoirs situés à l'intérieur, le dispositif étant adapté pour le stockage et
le dégagement contrôlé d'un médicament ;
de fournir dans les réservoirs un médicament sous forme de matrice monolithique solide poreuse ; et
de combiner avec la matrice du médicament au moins un matériau excipient qui remplit sensiblement les pores de la matrice du médicament pour former un composite médicament/excipient,
dans lequel le composite médicament/excipient, seul ou en combinaison avec un autre matériau excipient, remplit sensiblement chacun des réservoirs.

29. Procédé selon la revendication 28, dans lequel la matrice du médicament est tout d'abord prévue dans lesdits un ou plusieurs réservoirs puis le matériau excipient fluidifié est ajouté aux réservoirs.

30. Procédé selon la revendication 28, dans lequel la matrice du médicament est formée par un procédé comprenant :
la dissolution ou la dispersion d'un médicament dans un milieu liquide volatil pour former un premier fluide ;
la déposition d'une quantité du premier fluide dans chacun des réservoirs ; et
le séchage de ladite quantité par volatilisation du milieu liquide volatil pour produire la matrice monolithique solide du médicament dans les réservoirs.

31. Procédé selon la revendication 28, dans lequel ledit au moins un matériau excipient est dans un état en fusion lorsqu'il est combiné avec la matrice du médicament.

32. Procédé selon la revendication 28, dans lequel la matrice du médicament et ledit au moins un matériau excipient sont tout d'abord combinés ensemble à l'extérieur des réservoirs pour former un composite médicament/excipient, et le composite médicament/excipient est alors chargé dans les réservoirs.

33. Procédé selon la revendication 32, dans lequel le composite médicament/excipient est solidifié sous une forme préalable avant d'être chargé dans les réservoirs, chaque forme préalable étant conformée pour se loger dans l'un des réservoirs et remplir sensiblement celui-ci.

34. Procédé selon la revendication 32, dans lequel le composite médicament/excipient est extrudé par fusion dans les réservoirs.

35. Procédé selon la revendication 29, comprenant en outre la solidification du matériau excipient fluidifié.

36. Procédé selon la revendication 28, dans lequel le matériau excipient comprend une solution saturée du médicament.

37. Procédé selon l'une quelconque des revendications 28 à 36, dans lequel les réservoirs sont des microréservoirs.
